# EUROPÄISCHE PATENTANMELDUNG

(11) **EP 1 374 828 A1**
(43) Veröffentlichungstag der Anmeldung: **02.01.2004**
(21) Anmeldenummer: 03013423.3
(22) Anmeldetag: 20.06.2003
(51) Int. Cl.: A61K 6/083

(54) **Dentalmaterialien auf der Basis von Hydroxyalkylacrylamiden**

(30) Priorität: 26.06.2002 DE 10228540
(71) Anmelder: Ivoclar Vivadent AG, 9494 Schaan (LI)
(72) Erfinder: Moszner, Norbert Prof. Dr., 9492 Eschen (LI); Angermann, Jörg Dr., 6800 Feldkirch (AT); Rheinberger, Volker Dr., 9490 Vaduz (LI); Zeuner, Frank Dr., 9490 Vaduz (LI)
(74) Vertreter: UEXKÜLL & STOLBERG

(57) **Zusammenfassung**

Polymerisierbare Zusammensetzungen, die mindestens ein Hydroxyalkylacrylamid mit der Formel (I) enthalten 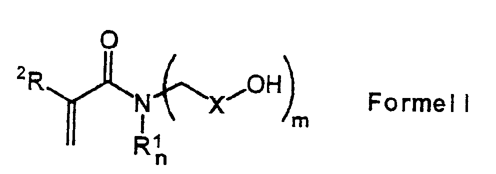 in der
- X: für einen C₁- bis C₁₂-Alkylenrest oder C₇- bis C₁₅-Alkylenphenylenrest steht,
- R¹: für einen C₁- bis C₁₀-Alkylrest, Phenyl oder Wasserstoff steht,
- m,n: unabhängig voneinander 0, 1 oder 2 sind, wobei m + n = 2, und
- R²: für Wasserstoff, Methyl oder X'-OH steht, wenn m ≥ 1, oder X'-OH steht, wenn m = 0, wobei X' eine der für X angegebenen Bedeutungen hat, und
wobei für n = 2 die beiden Reste R¹ und für m = 2 die beiden Reste -X- gleich oder verschieden sein können und die sich insbesondere als Dentalmaterialien eignen.

## Beschreibung

Die vorliegende Erfindung betrifft Zusammensetzungen auf der Basis von Hydroxyalkylacrylamiden, die sich durch eine hohe Hydrolysestabilität auszeichnen und sich besonders als Dentalmaterialien eignen.

In der Zahnmedizin werden Adhäsivsysteme gegenwärtig vor allem zur Herstellung eines festen und dauerhaften Verbunds zwischen Kompositen und der Zahnhartsubstanz (Schmelz und Dentin) eingesetzt. Unter Kompositen werden Dentalmaterialien verstanden, die ein polymerisierbares organisches Bindemittel und Füllstoff enthalten. Zur Verbesserung der Schmelzhaftung wird die Zahnschmelzoberfläche gewöhnlich mit 20 bis 40 %iger Phosphorsäure angeätzt, wodurch eine mikromechanische Verankerung der polymerisierten Adhäsivmonomere realisiert werden kann.

Dabei klassifiziert man die einzelnen Dentinadhäsivsysteme danach, ob die durch die Kavitätenpräparation gebildete Schmierschicht ganz oder teilweise aufgelöst wird, ohne jedoch weggespült zu werden, oder im Sinne der Totalätztechnik die Schmierschicht vollständig entfernt wird [vgl. U. Blunck, B. Haller, Quintessenz 50 (1999) 1021-1033].

Unabhängig von dieser Klassifikation enthalten zahlreiche Dentinadhäsive Hydroxyalkylmethacrylate und dabei vor allem 2-Hydroxyethylmethacrylat (HEMA), da dieses hydrophile Monomer selbst über ein ausgezeichnetes Penetrationsvermögen verfügt und gleichzeitig die Penetration anderer Monomere fördert (vgl. N. Nakabayashi und D. H. Pashley in "Hybridization of dental hard tissues", Quintessence Publishing Co, Tokyo etc, 1998, Seiten 11-13).

Darüber hinaus werden in selbstätzenden Dentinadhäsiven als Haftvermittler meist stark saure Monomere, vor allem polymerisationsfähige Phosphorsäureester, wie z. B. 10-Methacryloyloxydecyldihydrogenphosphat (MDP) und Dipentaerythritol-pentamethacryloyloxyphosphat (PENTA) eingesetzt.

Zur Verbesserung des Benetzungsvermögens enthalten viele Adhäsive neben HEMA und sauren Monomeren auch Wasser, was eine Spaltung von HEMA in Ethylenglycol und Methacrylsäure zur Folge hat, so daß diese Mischungen nicht hydrolyse- und lagerstabil sind. Neben einer Beeinträchtigung der Hafteigenschaften des entsprechenden Adhäsivs kommt es hierbei zu toxikologischen Problemen.

Während durch das Anätzen des Zahnschmelzes eine ausreichende Schmelzhaftung erzielt werden kann, ist eine Haftung am Dentin schwieriger erreichbar.

Gemäß der US 5,866,631 soll durch die Verwendung von dentalen Primern auf der Basis saurer Monomere, die eine Mischung von Arylboraten und Übergangsmetallverbindungen als Polymerisationskatalysatoren enthalten, eine hohe Haftung an Dentin und Zahnschmelz erzielt werden. Als photopolymerisierbares Monomer wird unter anderem Trimethylolpropantriacrylat aufgeführt.

Die US 5,058,264 schlägt zur Verbesserung der Dentinhaftung von Dentalmaterialien die Verwendung von (Meth)acrylsäureestern und (Meth)acrylamiden mit Formylpiperazingruppen vor. Diese Verbindungen können auf Grund ihrer Aldehydgruppen mit den primären Aminogruppen von Kollagen unter Ausbildung von Azomethinen reagieren und so die Dentinhaftung verbessern. Nachteilig ist die Hohe Reaktivität der Aldehydgruppe, die die Stabilität der Verbindungen beeinträchtigt und Nebenreaktionen mit anderen Komponenten von Dentalmaterialien begünstigt. Zudem wirken Aldehyde mutagen.

Die WO 98/01419 offenbart die Herstellung von N-(hydroxyalkyl) substituierten (Meth) acrylamiden durch Amidierung von α, ω-Hydroxyalkylaminen mit (Meth)acrylsäurechlorid, wie z. B. von N-(2-Hydroxyethyl)acrylamid, durch Umsetzung von Ethanolamin mit (Meth)acrylsäurechlorid. Diese Intermediate werden durch Reaktion der Hydroxylgruppe mit weiterem (Meth)acrylsäurechlorid zu multifunktionellen Vernetzern umgesetzt, die sich zur Herstellung von Trägergelen für die Gelelektrophorese eignen sollen (vgl. auch G. Y. N. Chan, M. G. Looney, D. H. Solomon, S. Veluayitham, Austr. J. Chem. 51 (1998)31-35).

Die WO 89/08099 offenbart die Herstellung von ungesättigten N-(2-Hydroxyalkyl)-2-alkyl-amiden durch Umsetzung von ungesättigten 2-Alkylestern mit β-Aminoalkanolen in Gegenwart von katalytischen Mengen von Alkalihydroxiden oder Alkalialkoxiden. Die Amide dienen als Edukte zur Herstellung von Vinyloxazolinen.

T. Fukushima, T. Itoh, Y. Inoue, M. Kawaguchi, K. Miyazaki, Journal of Dentistry, 27 (1999) 391-397, beschreiben Dentinprimer auf der Basis von N-Methylolacrylamid und N-Methylolmethacrylamid. N-Methylolverbindungen sind jedoch nicht stabil und neigen zur Abspaltung von Formaldehyd. N-Methylolacrylamid ist zudem kanzerogen, so daß diese Verbindung zur Herstellung von Dentalmaterialien wenig vorteilhaft ist.

Der Erfindung liegt die Aufgabe zugrunde, Dentalmaterialien bereitzustellen, die auch in Gegenwart von stark aciden Verbindungen hydrolysestabil sind und eine geringe Toxizität aufweisen. Zudem sollen die Materialien eine gute Haftung auf Dentin und Zahnschmelz zeigen.

Die Aufgabe wird erfindungsgemäß durch polymerisierbare Zusammensetzungen gelöst, die mindestens ein Hydroxyalkylacrylamid mit der allgemeinen Formel (I) enthalten 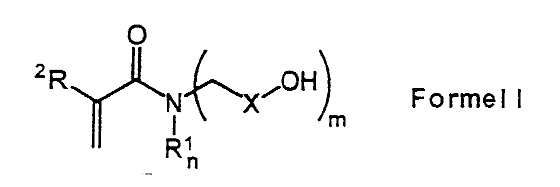 in der
- X: für einen C₁- bis C₁₂-Alkylenrest oder C₇- bis C₁₅-Alkylenphenylenrest, vorzugsweise für einen C₁- bis C₁₀-Alkylenrest und ganz besonders bevorzugt für einen C₁- bis C₈-Alkylenrest steht,
- R¹: für einen C₁- bis C₁₀-Alkylrest, Phenyl oder Wasserstoff steht, vorzugsweise für einen C₁- bis C₆-Alkylrest oder Wasserstoff, besonders bevorzugt einen C₁- bis C₃-Alkylrest oder Wasserstoff steht,
- m,n: unabhängig voneinander 0, 1 oder 2 sind, wobei m + n gleich 2 ist, und
- R²: für Wasserstoff, Methyl oder X'-OH steht, wenn m größer oder gleich 1 ist, oder für X'-OH steht, wenn m gleich 0 ist, wobei X' eine der für X angegebenen Bedeutungen hat und vorzugsweise C₁- bis C₆-Alkylen, besonders bevorzugt C₁- bis C₃-Alkylen ist, und
wobei für n = 2 die beiden Reste R¹ und für m = 2 die beiden Reste -X- gleich oder verschieden sein können. Die Hydroxyalkylacrylamide gemäß Formel (I) enthalten keine Aldehydgruppen, und vorzugsweise enthalten die Zusammensetzungen auch keine anderen Verbidungen mit Aldehydgruppen.

Bei den genannten Alkyl- und Alkylenresten handelt es sich vorzugsweise um lineare Gruppen. Verbindungen, bei denen m und n jeweils gleich 1 sind, sind besonders bevorzugt. Weiter bevorzugt sind Verbindungen, die 1, 2 oder 3 Hydroxylgruppen pro Molekül aufweisen.

Hydroxyalkylacrylamide gemäß Formel (I) sind analog zu WO 89/08099 zugänglich. Beispielsweise können N-(Hydroxyalkyl)methacrylamide (m ≥ 1, R² = CH₃) durch Amidierung von Methacrylsäureestern mit N-(Hydroxyalkyl)aminen erhalten werden:

### Allgemein:

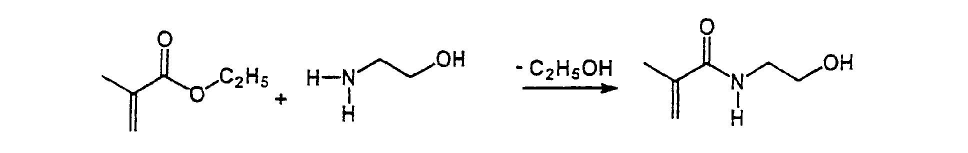

### Konkretes Beispiel:

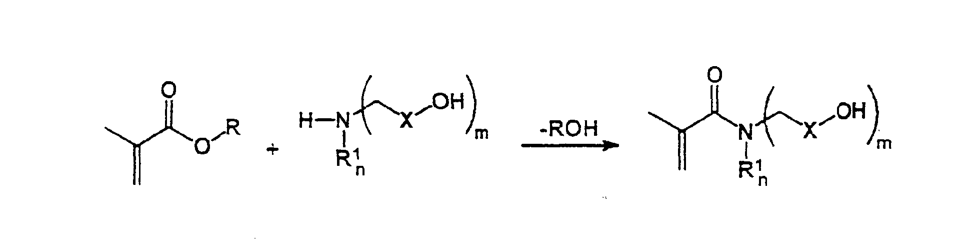

N-(Hydroxyalkyl)acrylamide (m ≥ 1, R² = H) können vorteilhafterweise analog zu WO 98/01419 durch Reaktionen von Acrylsäurechlorid bzw. -anhydrid mit Aminoalkoholen erhalten werden:

### Allgemein:

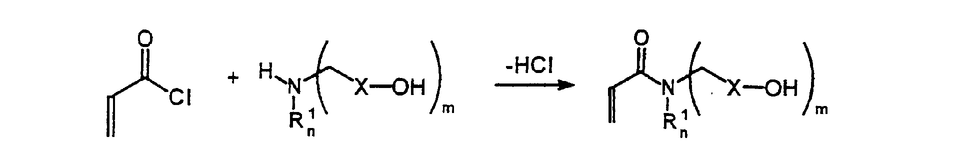

### Konkretes Beispiel:

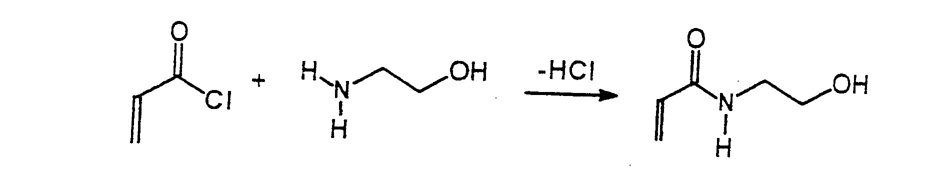

2-(Hydroxyalkyl)acrylamide (m = 0) sind durch die Hydroxyalkylierung von entsprechenden Acrylamiden analog zu E. Ciganek, The catalyzed α-Hydroxyalkylation and α-Aminoalkylation of activated olefines, in Organic Reactions, 51. Auflage, Seiten 223, 309 und 328 erhältlich, beispielsweise durch Umsetzung von N,N-Dimethylacrylamid mit Formaldehyd:

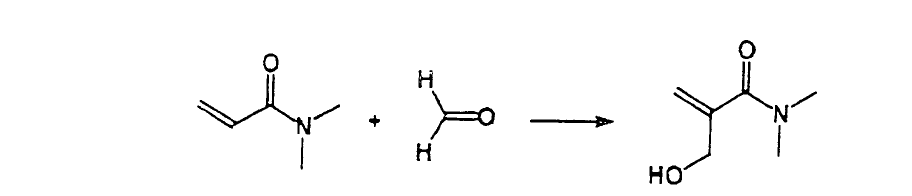

Verbindungen mit m = 2 werden analog denen mit m = 1 hergestellt, wobei man jedoch von den Di-(hydroxyalkyl)aminen ausgeht, die z.B. mit Acrylsäurechlorid acryliert werden.

Bevorzugte Beispiele für die erfindungsgemäßen Hydroxyalkyl)acrylamide der Formel (I) sind:

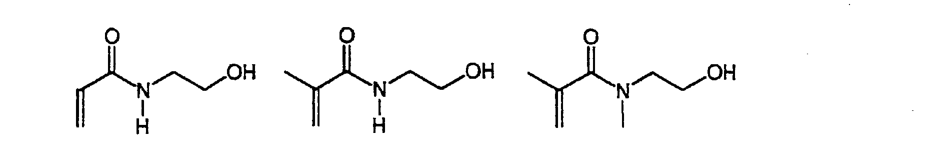

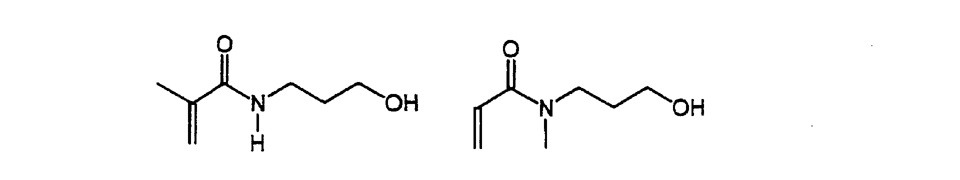

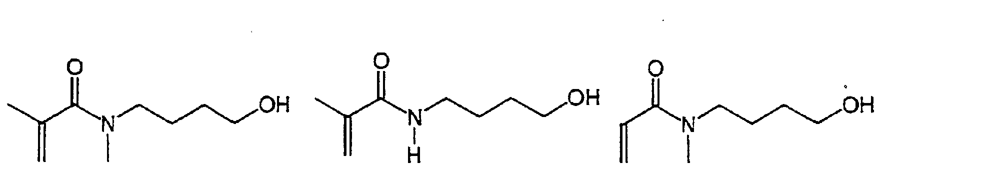

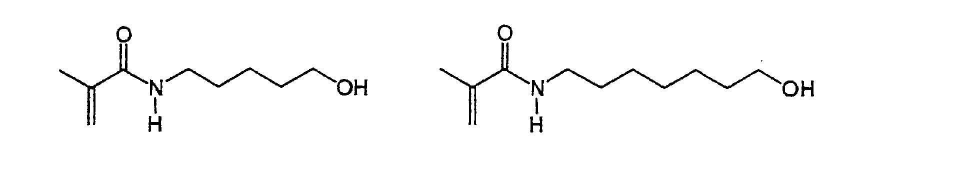

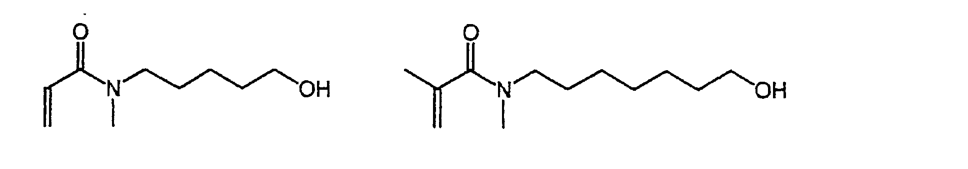

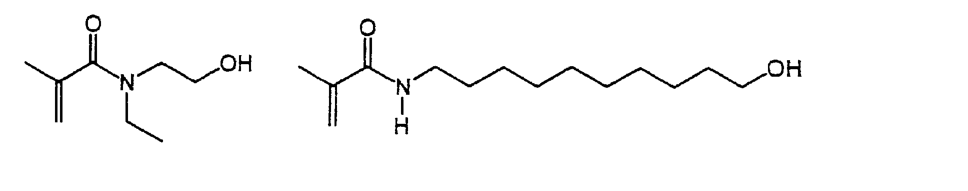

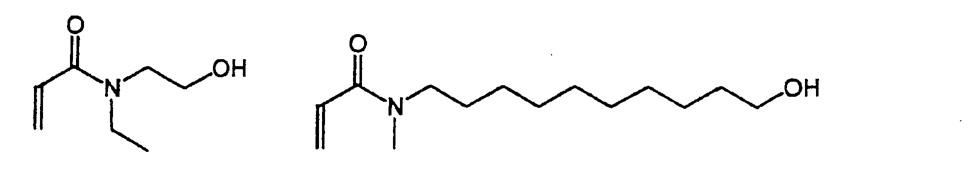

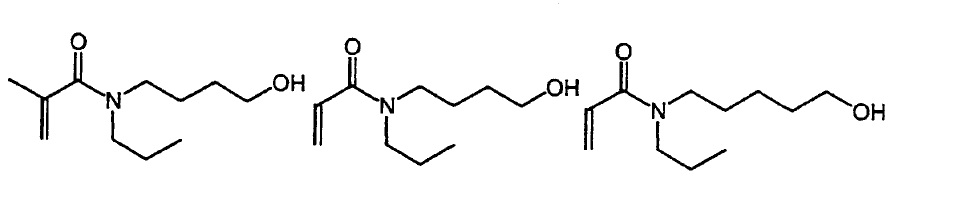

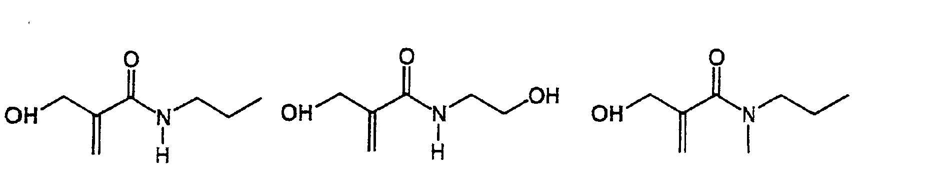

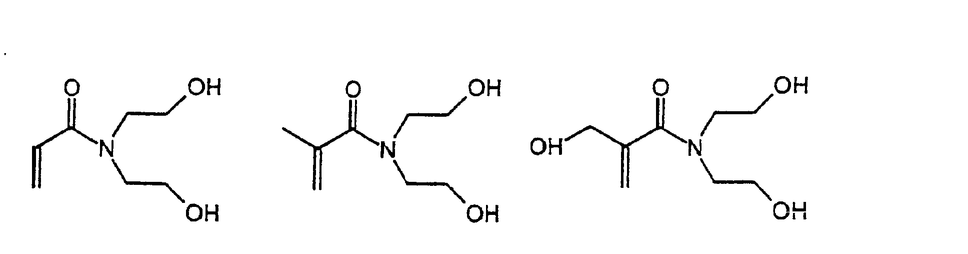

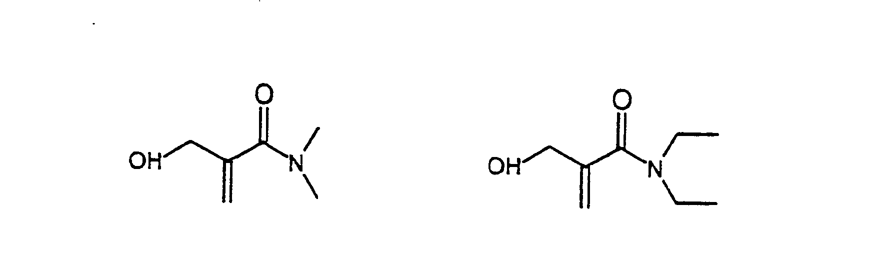

Neben dem Hydroxyalkylacrylamid gemäß Formel (I) enthalten die erfindungsgemäßen Zusammensetzungen vorzugsweise mindestens ein weiteres polymerisierbares Monomer. Als weitere polymerisierbare Monomere eignen sich besonders radikalisch polymerisierbare Monomere und kationisch polymerisierbare Monomere.

Die radikalisch polymerisierbaren Monomere können eine oder mehrere radikalisch polymerisierbare Gruppen aufweisen, wobei Zusammensetzungen, die mindestens ein Monomer mit 2 oder mehr, vorzugsweise 2 bis 5 radikalisch polymerisierbaren Gruppen enthalten, bevorzugt sind. Monomere mit 2 oder mehr polymerisierbaren Gruppen wirken als Vernetzer und erhöhen so die mechanische Stabilität der gehärteten Zusammensetzungen.

Bevorzugte Vernetzermonomere sind Bis-GMA, Glycerindimethacrylat, und besonders Urethane aus 2-(Hydroxymethyl)acrylsäure und Diisocyanaten, wie 2,2,4-Trimethylhexamethylendiisocyanat oder Isophorondiisocyanat, oder kommerziell zugängliche Bis(meth)-acrylamide, wie N,N'-Diethyl-1,3-bis(acrylamido)-propan, 2,6-Dimethylen-4-oxaheptan-1,7-dicarbonsäure-bis(propylamid), 1,6-Bis-(acrylamido)-2,2,4(2,4,4)-trimethylhexan und N,N'-Dimethyl-1,6-bis-(acrylamido)hexan, und die kommerziell zugänglichen Bis(meth)acrylamide, wie 1,4-Bisacryloylpiperazin, Methylenund Ethylenbisacrylamid.

Zusammensetzungen, die neben dem Hydroxyalkylacrylamid gemäß Formel (I) 1 bis 45 Gew.-%, vorzugsweise 5 bis 30 Gew.-% und ganz besonders bevorzugt 5 bis 20 Gew.-% Vernetzermonomer enthalten, insbesondere Bis(meth)acrylamid, sind erfindungsgemäß besonders bevorzugt. Diese und, wenn nicht anders angegeben, alle anderen Prozentangaben beziehen sich auf die Gesamtmasse der Zusammensetzung.

Als radikalisch polymerisierbare Monomere sind weiterhin bei Raumtemperatur flüssige Monomere zu nennen, die sich als Verdünnermonomere eignen. Bevorzugt sind Monomere mit einer Viskosität von 0,01 bis 10 Pa·s bei Raumtemperatur. Besonders bevorzugt sind Mono(meth)acrylate und auf Grund ihrer Hydrolysestabilität insbesondere Mesitylmethacrylat und 2-(Alkoyxymethyl)acrylsäuren, z. B. 2-(Ethoxy-methyl)acrylsäure, und Allylether. Weitere bevorzugte hydrolysestabile Monomere sind N-Vinylpyrrolidon und N,N-dialkylsubstituierte Acrylamide, wie z.B. das kommerziell zugängliche, dünnflüssige N,N-Dimethylacrylamid. Unter Alkyl werden bevorzugt Reste mit 1 bis 6, insbesondere 1 bis 3 Kohlenstoffatomen verstanden.

Die erfindungsgemäßen Zusammensetzungen enthalten neben dem Hydroxyalkylacrylamid gemäß Formel (I) vorzugsweise 0 bis 50 Gew.-%, vorzugsweise 10 bis 40 Gew.-% und ganz besonders bevorzugt 10 bis 30 Gew.-% Verdünnungsmonomer.

Gemäß einer weiteren bevorzugten Ausführungsform enthalten die Zusammensetzungen mindestens ein acides radikalisch polymerisierbare Monomer, d.h. ein Monomer mit einer oder mehreren aciden Gruppen, wie Carbonsäureanhydrid-, Carbonsäure-, Phosphorsäure-, Dihydrogenphosphat-, Phosphonsäure- und Sulfonsäuregruppen. Bevorzugte acide Gruppen sind Phosphorsäure- und Phosphonsäuregruppen. Besonders bevorzugte acide Monomere sind 4-(Meth)-acryloyloxyethyltrimellitsäureanhydrid,10-Methacryloyloxydecylmalonsäure, N-(2-Hydroxy-3-methacryloyloxypropyl)-N-phenylglycin, 4-Vinylbenzoesäure, 2-Methacryloyloxyethyl-phenyl-hydrogenphosphat, PENTA, MDP, Vinylsulfonsäure und 4-Vinylbenzolsulfonsäure. Auf Grund ihrer Hydrolysestabilität sind 4-Vinylbenzylphosphonsäure und 2 - [4- (Dihydroxyphosphoryl) -2-oxa-butyl] -acrylsäure (DHPOBAE) sowie deren Amide und hydrolysestabile Ester, wie z.B. der Mesitylester (= 2,4,6-Triphenylester), besonders bevorzugt.

Zusammensetzungen, die neben dem Hydroxyalkylacrylamid gemäß Formel (I) 1 bi 50 Gew.-%, vorzugsweise 5 bis 40 Gew.-% und ganz besonders bevorzugt 10 bis 30 Gew.-% acides Monomer enthalten, insbesondere acides Monomer mit Dihydrogenphosphat-, Phosphonsäure- und/oder Sulfonsäuregruppen, sind erfindungsgemäß besonders bevorzugt.

Als kationisch polymerisierbare Monomere eignen sich besonders die kommerziell zugänglichen Vinylether und Oxetane.

Die genannten Monomere können jeweils allein oder als Mischung eingesetzt werden. Erfindungsgemäß sind besonders solche Monomere bevorzugt, die eine hohe Hydrolysestabilität aufweisen. Im Zusammenhang mit der vorliegenden Erfindung werden solche Verbindungen als hydrolysestabil bezeichnet, die in Wasser oder in Mischungen von Wasser und wassermischbaren Lösungsmitteln in Gegenwart starker Säuren, wie z.B. 20 - 30 %iger Phosphorsäure, bei 37°C für mindestens 4 Wochen stabil sind. Diese Hydrolysestabilität zeigen vor allem polymerisierbare Carbonsäureamide und Mesitylester, deren Esterhydrolyse sterisch gehemmt ist.

Zur Initiierung der Polymerisation enthalten die erfindungsgemäßen Zusammensetzungen vorzugsweise einen Initiator für die radikalische Polymerisation, besonders bevorzugt einen Initiator für die Photopolymerisation. Hierzu eignen sich insbesondere Benzophenon, Benzoin sowie deren Derivate oder α-Diketone oder deren Derivate, wie 9,10-Phenanthrenchinon, Diacetyl oder 4,4-Dichlorbenzil. Besonders bevorzugt werden Campherchinon und 2,2-Methoxy-2-phenyl-acetophenon und ganz besonders bevorzugt α-Diketone in Kombination mit Aminen als Reduktionsmittel eingesetzt. Bevorzugte Amine sind 4-(Dimethylamino)-benzoesäureester, N, N-Dimethylaminoethylmethacrylat, N,N-Dimethyl-sym.-xylidin und Triethanolamin. Darüber hinaus sind auch Acylphosphinoxide, wie z. B. 2,4,6-Trimethylbenzoyldiphenylphosphinoxid oder Bis(2,6-dichlorbenzoyl)-4-N-propylphenylphosphinoxid geeignet.

Für die Aushärtung von kationisch polymerisierbare Monomere enthaltenden Zusammensetzungen eignen sich besonders Diaryliodonium- oder Triarylsulfoniumsalze, wie z. B. Triphenylsulfoniumhexafluorophosphat oder -hexafluoroantimonat. Dabei sind solche Initiatoren bevorzugt, die auch die für die Aushärtung der Hydroxyalkylacrylamide erforderlichen Radikale bilden, wie z.B. Diphenyliodoniumhexafluoroantimonat und insbesondere Diphenyliodoniumtetra(pentafluorophenyl)borat.

Weiterhin können die erfindungsgemäß eingesetzten Zusammensetzungen zur Verbesserung der mechanischen Eigenschaften Füllstoff enthalten. Als Füllstoffe eignen sich organische und anorganische Partikel und Fasern. Bevorzugte anorganische partikuläre Füllstoffe sind amorphe kugelförmige Materialien auf der Basis von Metalloxiden, wie ZrO₂ und TiO₂ oder Mischoxiden aus SiO₂, ZrO₂ und/oder TiO₂; nanopartikuläre oder mikrofeine Füllstoffe, wie pyrogene Kieselsäure oder Fällungskieselsäure; monodisperse, nanopartikuläre Füllstoffe auf Basis von SiO₂, ZrO₂, Al₂O₃, AlO(OH) oder deren Mischoxiden, vorzugsweise mit einer mittleren Partikelgröße von 5 bis 200 nm, besonders bevorzugt 10 bis 100 nm, ganz besonders bevorzugt 10 bis 50 nm; sowie mikrofeine Füllstoffe, wie Quarz-, Keramik-, Glaskeramik- oder Glaspulver, mit einer mittleren Teilchengröße von 0,01 bis 5 µm, vorzugsweise 0,3 bis 5 µm, besonders bevorzugt 0,4 bis 3 µm und ganz besonders bevorzugt 0,4 bis 1 µm; sowie röntgenopake Füllstoffe, wie Ytterbiumtrifluorid.

Darüber hinaus können die erfindungsgemäßen Zusammensetzungen weitere Additive enthalten, vor allem Lösungsmittel, wie Wasser, Ethylacetat und Ethanol, bzw. Lösungsmittelgemische, Stabilisatoren, Aromastoffe, mikrobiocide Wirkstoffe, fluoridionenabgebende Additive, optische Aufheller, Weichmacher und UV-Absorber.

Die erfindungsgemäßen Zusammensetzungen eignen sich besonders als Dentalmaterialien, insbesondere als Adhäsive und Zemente, wie z.B. Befestigungszemente. Solche Adhäsive zeichnen sich durch eine sehr gute Haftung auf der Zahnhartsubstanz, d.h. auf Schmelz und Dentin, aus und sind in Gegenwart von Wasser oder Alkohol hydrolyse- und alkoholysestabil.

Erfindungsgemäß sind solche Dentalmaterialien bevorzugt, die die folgenden Komponenten enthalten:
a) 0,5 bis 70 Gew.-%, bevorzugt 10 bis 60 Gew.-% und besonders bevorzugt 20 bis 50 Gew.-% Hydroxyalkylacrylamid gemäß Formel (I),
b) 0,01 bis 5 Gew.-%, besonders bevorzugt 0,1 bis 0,2 Gew.-% Initiator für die radikalische Polymerisation,
c) 0 bis 80 Gew.-%, bevorzugt 0 bis 60 Gew.-% und besonders bevorzugt 0 bis 50 Gew.-% weitere polymerisierbare Monomere,
d) 0 bis 95 Gew.-%, bevorzugt 0 bis 80 Gew.-% und besonders bevorzugt 0 bis 40 Gew.-% Lösungsmittel, vorzugsweise Wasser,
e) 0 bis 75 Gew.-% Füllstoff.

Die erfindungsgemäßen Zusammensetzungen eignen sich besonders als Dentalmaterialien, ganz besonders als dentale Adhäsive oder Zemente. Die bevorzugte Zusammensetzung der Materialien richtet sich nach dem gewünschten Verwendungszweck.

Bevorzugte Adhäsive enthalten die folgenden Komponenten:
a) 0,5 bis 70 Gew.-%, bevorzugt 10 bis 60 Gew.-% und besonders bevorzugt 20 bis 50 Gew.-% Hydroxyalkylacrylamid gemäß Formel (I) ,
b) 0,01 bis 5 Gew.-%, besonders bevorzugt 0,1 bis 0,2 Gew.-% Initiator für die radikalische Polymerisation,
c) 0 bis 80 Gew.-%, bevorzugt 0 bis 60 Gew.-% und besonders bevorzugt 0 bis 50 Gew.-% weitere polymerisierbare Monomere,
d) 0 bis 40 Gew.-%, bevorzugt 10 bis 30 Gew.-% und besonders bevorzugt 10 bis 25 Gew.-% Lösungsmittel, vorzugsweise Wasser,
e) 0 bis 60 Gew.-%, bevorzugt 0 bis 50 Gew.-% und besonders bevorzugt 0 bis 30 Gew.-% Füllstoff, vorzugsweise monodisperse, nanopartikuläre Füllstoffe auf Basis von SiO₂, ZrO₂, Al₂O₃, AlO(OH) oder deren Mischoxiden, vorzugsweise mit der oben definierten Partikelgröße.

Bevorzugte Zemente enthalten die folgenden Komponenten:
a) 0,5 bis 70 Gew.-%, bevorzugt 10 bis 60 Gew.-% und besonders bevorzugt 20 bis 50 Gew.-% Hydroxyalkylacrylamid gemäß Formel (I),
b) 0,01 bis 5 Gew.-%, besonders bevorzugt 0,1 bis 0,2 Gew.-% Initiator für die radikalische Polymerisation,
c) 0 bis 80 Gew.-%, bevorzugt 0 bis 60 Gew.-% und besonders bevorzugt 0 bis 50 Gew.-% weitere polymerisierbare Monomere,
d) 1 bis 75 Gew.-%, vorzugsweise 5 bis 75 Gew.-% und besonders bevorzugt 40 bis 65 Gew.-% Füllstoff, vorzugsweise mikrofeine Füllstoffe auf Basis von Quarz-, Keramik-, Glaskeramikoder Glaspulver, mit der oben definierten Teilchengröße.

Alle Prozentangaben beziehen sich auf die Gesamtmasse der Zusammensetzung. Als weitere polymerisierbare Monomere werden vorzugsweise eines oder mehrere der oben definierten Monomere eingesetzt, besonders bevorzugt in den dort jeweils definierten Mengen, wobei die Mengen der einzelnen Monomere so gewählt werden, daß die Gesamtmenge an zusätzlichem Monomer die oben definierten Bereiche nicht überschreitet. Ganz besonders bevorzugt sind Zusammensetzungen, die mindestens ein acides Monomer oder mindestens ein Vernetzermonomer, insbesondere mindestens ein acides Monomer und mindestens ein Vernetzermonomer enthalten.

Die bevorzugten erfindungsgemäßen Zusammensetzungen härten unter Ausbildung von stark vernetzten Polymernetzwerken aus, die in Wasser wenig oder gar nicht quellen.

Die Erfindung wird im folgenden anhand von Beispielen näher erläutert.

### Ausführungsbeispiele

### Beispiel 1: Untersuchung der Hydrolysestabilität von N-(2-Hydroxyethyl)-N-methyl-acrylamid (HEMAM)

Es wurde eine 20%ige Lösung von HEMAM, das durch Umsetzung von N-Methylethanolamin mit Acrylsäurechlorid erhalten wurde, (stabilisiert mit 200 ppm 2, 6-Di-t-butyl-4-methylphenol) in D₂O/EtOHd₆/H₃PO₄ (2:2:1) hergestellt, diese bei 37 °C gelagert und ¹H-NMRspektroskopisch untersucht. Nach einer Standzeit von 3 Monaten konnten keine Veränderungen des ¹H-NMR-Spektrums festgestellt werden.

### Beispiel 2: Herstellung eines Adhäsivs auf der Basis von HEMAM

Zur Untersuchung der Dentinhaftung auf Rinderzahndentin wurden zwei Adhäsive folgender Zusammensetzung (Angabe in Gew.-%) hergestellt, die entweder HEMAM oder HEMA (Vergleichsbeispiel) enthielten:

| | |
|---|---|
| HEMAM oder HEMA: | 20,0 % |
| Glycerindimethacrylat: | 10,0 % |
| DHPOBAE¹⁾ : | 11, 1 % |
| Ethanol: | 25,0 % |
| Bis-GMA:²⁾ | 33.1 % |
| Photoinitiator³⁾: | 0,8 % |

| | |
|---|---|
| ¹⁾ DHPOBAE = 2-[4-(Dihydroxyphosphoryl)-2-oxa-butyl]-acrylsäure- ethylester | |
| ²⁾ Bis-GMA = Additionsprodukt aus Methacrylsäure und Bisphenol-A-diglycidylether | |
| ³⁾ Mischung aus Campherchinon (0,2 Gew.-%), 4-(N,N-Dimethylaminobenzoesäureethylester (0,3 Gew.-%) und dem Acylphosphinoxid Lucerin TPO (0,3 Gew.-%, BASF) | |

Rinderzähne wurden so in Kunststoffzylinder eingebettet, daß sich das Dentin und der Kunststoff in einer Ebene befanden. Nach 15 s Ätzen mit 37 %-iger Phosphorsäure wurde gründlich mit Wasser gespült. Durch die Säureätzung wurden die Dentintubuli geöffnet. Dann wurde mit einem kleinen Pinsel eine Schicht Adhäsiv obiger Zusammensetzung aufgebracht, mit dem Luftbläser zur Entfernung des Lösungsmittels kurz verblasen und für 40 s mit einer Halogenlampe (Astralis® 7, Vivadent) belichtet. Auf die Adhäsivschicht wurde ein Zylinder aus einem handelsüblichen Kompositmaterial (Tetric® Ceram, Vivadent) in zwei Schichten von je 1-2 mm aufpolymerisiert.

Anschließend wurden die Prüfkörper für 24 h bei 37 °C in Wasser gelagert und die Scherhaftfestigkeit gemäß der ISO-Richtlinie "ISO 1994-ISO TR 11405: Dental materials Guidance on testing of adhesion to tooth structure" bestimmt. Dabei ergab sich für das Adhäsiv mit HEMAM eine Scherhaftfestigkeit von 16,95 Mpa, für das Adhäsiv mit HEMA von 20,02 MPa. Die Ergebnisse zeigen, daß die erfindungsgemäß verwendeten Hydroxyalkylacrylamide bei deutlich verbesserter Hydrolysestabilität (vgl. Beispiel 1) eine mit HEMA vergleichbare Scherhaftfestigkeit ergeben.

## Patentansprüche

1. Polymerisierbare Zusammensetzung, **dadurch gekennzeichnet, daß** sie mindestens ein Hydroxyalkylacrylamid mit der Formel (I) enthält 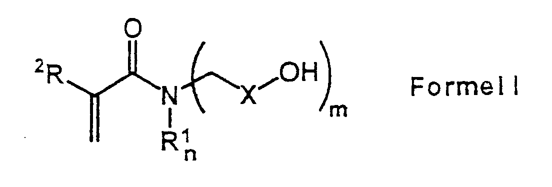 in der
X für einen C₁- bis C₁₂-Alkylenrest oder C₇- bis C₁₅-Alkylenphenylenrest steht,
R¹ für einen C₁- bis C₁₀-Alkylrest, Phenyl oder Wasserstoff steht,
m,n unabhängig voneinander 0, 1 oder 2 sind, wobei m + n = 2, und
R² für Wasserstoff, Methyl oder X'-OH steht, wenn m ≥ 1, oder X'-OH steht, wenn m = 0, wobei X' eine der für X angegebenen Bedeutungen hat, und
wobei für n = 2 die beiden Reste R¹ und für m = 2 die beiden Reste -X- gleich oder verschieden sein können.

2. Zusammensetzung nach Anspruch 1, **dadurch gekennzeichnet, daß**
X für einen C₁- bis C₁₀-Alkylenrest steht,
R¹ für einen C₁- bis C₆-Alkylrest oder Wasserstoff steht,
R² für Wasserstoff, Methyl oder X'-OH steht, wenn m ≥ 1, oder X'-OH steht, wenn m = 0, wobei X' C₁- bis C₆-Alkylen ist.

3. Zusammensetzung nach Anspruch 1, **dadurch gekennzeichnet, daß** sie neben dem Hydroxyalkylacrylamid mindestens ein weiteres polymerisierbares Monomer und/oder einen Initiator für die radikalische Polymerisation enthält.

4. Zusammensetzung nach Anspruch 3, **dadurch gekennzeichnet, daß** sie mindestens ein radikalisch polymerisierbares Monomer enthält.

5. Zusammensetzung nach Anspruch 4, **dadurch gekennzeichnet, daß** sie mindestens ein Monomere mit 2 oder mehr polymerisierbaren Gruppen und/oder mindestens ein Monomer mit einer oder mehreren aciden Gruppen enthält.

6. Zusammensetzung nach einem der Ansprüche 3 bis 5, **dadurch gekennzeichnet, daß** sie mindestens ein kationisch polymerisierbares Monomer enthält.

7. Zusammensetzung nach einem der Ansprüche 1 bis 6, **dadurch gekennzeichnet, daß** sie einen Initiator für die Photopolymerisation enthält.

8. Zusammensetzung nach einem der Ansprüche 1 bis 7, **dadurch gekennzeichnet, daß** sie Füllstoff enthält.

9. Zusammensetzung nach einem der Ansprüche 1 bis 8, **dadurch gekennzeichnet, daß** sie
0,5 - 70 Gew.-% Hydroxyalkylacrylamid,
0,01 - 5 Gew.-% Polymerisationsinitiator,
0 - 80 Gew.-% weiteres polymerisierbares Monomer und
0 - 95 Gew.-% Lösungsmittel
enthält.

10. Zusammensetzung nach Anspruch 9, **dadurch gekennzeichnet, daß** sie
a) 0,5 bis 70 Gew.-% Hydroxyalkylacrylamid gemäß Formel (I) ,
b) 0,01 bis 5 Gew.-% Initiator für die radikalische Polymerisation,
c) 0 bis 80 Gew.-% weitere polymerisierbare Monomere,
d) 0 bis 40 Gew.-% Lösungsmittel,
e) 0 bis 60 Gew.-% Füllstoff enthält.

11. Zusammensetzung nach Anspruch 10, **dadurch gekennzeichnet, daß** sie monodispersen, nanopartikulären Füllstoff auf Basis von SiO₂, ZrO₂, Al₂O₃, AlO(OH) oder deren Mischoxiden enthält.

12. Zusammensetzung nach Anspruch 10 oder 11, **dadurch gekennzeichnet, daß** der Füllstoff eine Partikelgröße von 5 bis 200 nm aufweist.

13. Zusammensetzung nach Anspruch 9, **dadurch gekennzeichnet, daß** sie
a) 0,5 bis 70 Gew.-% Hydroxyalkylacrylamid gemäß Formel (I),
b) 0,01 bis 5 Gew.-% Initiator für die radikalische Polymerisation,
c) 0 bis 80 Gew.-% weitere polymerisierbare Monomere,
d) 1 bis 75 Gew.-% Füllstoff enthält.

14. Zusammensetzung nach Anspruch 13, **dadurch gekennzeichnet, daß** sie mikrofeine Füllstoffe auf Basis von Quarz-, Keramik-, Glaskeramik- oder Glaspulver enthält.

15. Zusammensetzung nach Anspruch 13 oder 14, **dadurch gekennzeichnet, daß** der Füllstoff eine mittlere Teilchengröße von 0,01 bis 5 µm aufweist.

16. Zusammensetzung nach einem der Ansprüche 9 bis 15, **dadurch gekennzeichnet, daß** sie als weiteres polymerisierbares Monomer 1 bis 50 Gew.-% Monomer mit einer oder mehreren aciden Gruppen enthält.

17. Zusammensetzung nach einem der Ansprüche 9 bis 16, **dadurch gekennzeichnet, daß** sie als weiteres polymerisierbares Monomer 1 bis 45 Gew.-% Monomer mit 2 oder mehr polymerisierbaren Gruppen enthält.

18. Verwendung einer Zusammensetzung gemäß einem der Ansprüche 1 bis 17 als Dentalmaterial.

19. Verwendung nach Anspruch 18 als Adhäsiv oder Zement.

20. Verwendung von einem oder mehreren Hydroxyalkylacrylamiden mit der Formel (I) 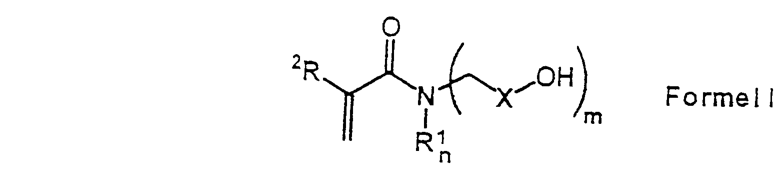 in der
X für einen C₁- bis C₁₂-Alkylenrest oder C₇- bis C₁₅-Alkylenphenylenrest steht,
R¹ für einen C₁- bis C₁₀-Alkylrest, Phenyl oder Wasserstoff steht,
m,n unabhängig voneinander 0, 1 oder 2 sind, wobei m + n = 2, und
R² für Wasserstoff, Methyl oder X'-OH steht, wenn m ≥ 1, oder X'-OH steht, wenn m = 0, wobei X' eine der für X angegebenen Bedeutungen hat, und
wobei für n = 2 die beiden Reste R¹ und für m = 2 die beiden Reste -X- gleich oder verschieden sein können, zur Herstellung eines Dentalmaterials.

21. Verwendung nach Anspruch 20, zur Herstellung eines Adhäsivs oder Zements.
